# FASCICULE DE BREVET EUROPEEN

(11) **EP 2 012 793 B1**
(45) Date de publication et mention de la délivrance du brevet: **08.04.2015**
(21) Numéro de dépôt: 07731937.4
(22) Date de dépôt: 24.04.2007
(51) Int. Cl.: A61K 31/55, A61K 31/5415, A61K 31/382, A61K 31/137, A61P 21/00, A61K 31/138

(54) **UTILISATION D'UN COMPOSE TRICYCLIQUE POUR PREPARER UN MEDICAMENT POUR TRAITER DES DEGENERESCENCES DU MUSCLE SQUELETTIQUE D'ORIGINE GENETIQUE**
VERWENDUNG EINER TRICYCLISCHEN VERBINDUNG ZUR HERSTELLUNG EINES ARZNEIMITTELS ZUR BEHANDLUNG VON SKELETTMUSKELDEGENERATION VON GENETISCHEM URSPRUNG
USE OF A TRICYCLIC COMPOUND FOR PREPARING A MEDICAMENT FOR TREATING SKELETAL MUSCLE DEGENERATION OF GENETIC ORIGIN

(30) Priorité: 28.04.2006 FR 0651516; 06.06.2006 FR 0652029
(43) Date de publication de la demande: 14.01.2009
(73) Titulaire: Association Française contre les Myopathies, 91002 Evry (FR)
(72) Inventeur: SEGALAT, Laurent, F-69340 Francheville (FR)
(74) Mandataire: Sarlin, Laure V.
(86) Numéro de dépôt international: PCT/FR2007/051164
(87) Numéro de publication internationale: WO 2007/125246

(56) Documents cités:
- CA-A- 2 292 874
- WANG ET AL.: "Lack of effects by tricyclic antidepressant and serotoinin inhibitors on anorexia in MCG 101 tumor-bearing mice with eicosanoid-related cachexia" NUTRITION, vol. 19, 2003, pages 47-53, XP002412735
- MOXLEY ET AL.: "Practice parameter: corticosteroid treatment of Duchenne dystrophy" NEUROLOGY, vol. 64, 2005, pages 13-20, XP002412736

## Description

La présente invention a pour objet l'utilisation de composés tricycliques, pour la préparation d'un médicament destiné à traiter des pathologies correspondant à une dégénérescence du muscle squelettique d'origine génétique.

La publication Nutrition, 2003, 19: 47-53 rapporte l'absence d'effet d'antidépresseurs tricycliques et d'inhibiteurs de la sérotonine sur l'anorexie, chez des souris MCG 101 porteuses de tumeurs avec une cachexie liée aux eicosanoides.

Les myopathies dégénératives (ou atrophies musculaires) d'origine génétique sont des maladies héréditaires dues à des mutations dans des gènes codant pour des protéines musculaires. Il existe plusieurs dizaines de myopathies dégénératives d'origine génétique. Bien que le tableau clinique puisse varier entre deux maladies, elles ont en commun de conduire inexorablement à l'atrophie musculaire des muscles dits squelettiques. Cette atrophie musculaire (aussi appelée fonte musculaire) se traduit par un handicap de plus en plus important pour le patient, qui peut conduire à la mort lorsque les muscles respiratoires sont atteints. La plus connue d'entre les myopathies dégénératives d'origine génétique est la myopathie de Duchenne (DMD). Il n'existe pas à ce jour de traitement efficace contre ces maladies.

Les cachexies, myopathies dégénératives (ou atrophies musculaires), non génétiques correspondent aux maladies conduisant à une fonte musculaire, indépendamment de l'atteinte primaire. La cachexie est notamment observée dans la plupart des cancers. Il n'existe pas de traitement efficace contre la cachexie cancéreuse.

Il existe donc un besoin pour de nouveaux moyens de traiter ces maladies et pour de nouveaux médicaments adaptés à de tels traitements.

Dans ce contexte et de façon totalement inattendue, l'inventeur de la présente demande de brevet a mis en évidence que certains composés tricycliques, dont certains sont déjà connus pour leur activité thérapeutique, et notamment, leur activité d'antidépresseur, permettaient de réduire la fonte des muscles squelettiques.

Par conséquent, la présente invention a pour objet l'utilisation pour la préparation d'un médicament destiné à traiter ou prévenir des pathologies correspondant à une dégénérescence musculaire des muscles squelettiques, d'origine génétique d'un composé de formule (I): 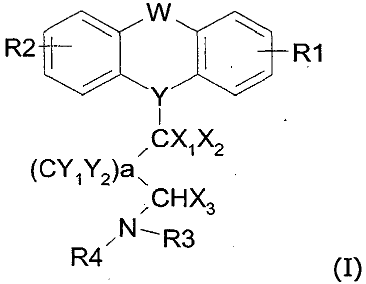 dans laquelle :
- R1 et R2 représentent, chacun indépendamment l'un de l'autre, un atome d'hydrogène ou un atome d'halogène, et en particulier un atome de chlore, ou un groupe trifluorométhyle, un groupe alkyle comprenant de 1 à 5 atomes de carbone, ou un groupe O-alkyle comprenant de 1 à 5 atomes de carbone,
- W représente un atome de soufre ou un enchaînement CH₂-CH₂ ou CH=CH, ou encore un groupe CH lié à Y pour former un pont (CH₂)₂,
- Y représente un atome d'azote ou un atome de carbone, étant entendu que lorsque W et Y sont liés par une chaîne (CH₂)₂, Y est atome de carbone,
- X1 représente un atome d'hydrogène ou un groupe alkyle de 1 à 4 atomes de carbone, ou bien X1 est lié avec R3 pour former un groupe pipéridine,
- X2 est défini comme suit :
   lorsque Y représente un atome d'azote ou un atome de carbone lié à W pour former un pont (CH₂)₂, X2 représente un atome d'hydrogène ou un groupe alkyle comprenant de 1 à 4 atomes de carbone,
   ou lorsque Y représente un atome de carbone non lié à W pour former un pont (CH₂)₂, X2 forme une liaison covalente entre le carbone auquel il est lié et Y, de sorte que Y-CX1X2 représente C=CX1,
- a est égal à 0 ou 1,
- Y1 et Y2 représentent, chacun indépendamment l'un de l'autre, un atome d'hydrogène ou un groupe alkyle de 1 à 4 atomes de carbone,
- X3 représente un atome d'hydrogène ou un groupe alkyle de 1 à 4 atomes de carbone, ou X3 est lié avec R3 pour former un groupe pipéridine,
- R3 et R4 sont définis comme suit :
   ∘ R3 représente un groupe alkyle de 1 à 5 atomes de carbone,
      ou bien R3 est lié avec X1 pour former un groupe pipéridine,
      ou bien R3 est lié avec X3 pour former un groupe pipéridine,
   o R4 représente un atome d'hydrogène ou un groupe alkyle de 1 à 5 atomes de carbone,
   ∘ ou bien R3 est lié à R4 pour former un groupe pipéridine, pyrrolidine, ou pipérazine dont l'atome d'azote est éventuellement substitué par un groupe alkyle de 1 à 4 atomes de carbone ou par un groupe-(CH₂)_{b}OH avec b qui est égal à 1, 2 ou 3,
éventuellement sous la forme de sels, solvats ou hydrates pharmaceutiquement acceptables.

Par alkyle, on entend, lorsqu'il n'est pas donné plus de précision, un radical hydrocarboné saturé, linéaire ou ramifié. A titre d'exemples de groupe alkyle, on pourra citer les groupes méthyle, éthyle, n-propyle, isopropyle, n-butyle, isobutyle, sec-butyle, t-butyle, n-pentyle.

Par halogène, on entend un atome de brome, iode ou fluor, et en particulier de chlore.

En particulier, on utilisera les composés de formule (I) ci-dessus, éventuellement sous la forme de sels, solvats ou hydrates pharmaceutiquement acceptables, dans lesquels :
- R1 et R2 représentent, chacun indépendamment l'un de l'autre, un atome d'hydrogène ou un atome d'halogène, et en particulier un atome de chlore, ou un groupe méthyle, trifluorométhyle ou méthoxy,
- W représente un atome de soufre ou un enchaînement CH₂-CH₂ ou CH=CH, ou encore un groupe CH lié à Y pour former un pont (CH₂)₂,
- Y représente un atome d'azote ou un atome de carbone, étant entendu que lorsque W et Y sont liés par une chaîne (CH₂)₂, Y est atome de carbone,
- X1 représente un atome d'hydrogène,
- X2 est défini comme suit :
   lorsque Y représente un atome d'azote ou un atome de carbone lié à W pour former un pont (CH₂)₂, X2 représente un atome d'hydrogène,
   ou lorsque Y représente un atome de carbone non lié à W pour former un pont (CH2)2, X2 forme une liaison covalente entre le carbone auquel il est lié et Y, de sorte que Y-CX1X2 représente C=CX1,
- a est égal à 0 ou 1,
- Y1 représente un atome d'hydrogène et Y2 représente un atome d'hydrogène ou un groupe méthyle,
- X3 représente un atome d'hydrogène ou un groupe méthyle, ou X3 est lié avec R3 pour former un groupe pipéridine,
- R3 et R4 sont définis comme suit :
   o R3 représente un groupe méthyle,
      ou bien R3 est lié avec X3 pour former un groupe pipéridine,
   o R4 représente un atome d'hydrogène ou un groupe méthyle,
   ∘ ou bien R3 est lié à R4 pour former un groupe pipéridine, ou pipérazine dont l'atome d'azote est éventuellement substitué par un groupe méthyle ou par un groupe -(CH2)_{b}OH avec b qui est égal à 2.

Si l'on numérote le carbone du groupe phényle comme suit : 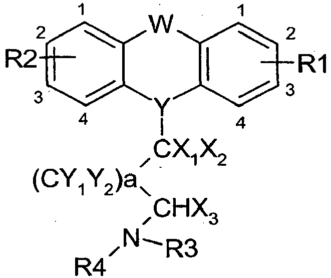 de préférence R1 et R2 se trouvent, chacun indépendamment l'un de l'autre, en position 2 ou 3 du groupe phényle auquel ils sont liés.

De préférence, les composés de formule (I) présentent une ou plusieurs des caractéristiques ci-dessous :
- a est égal à 1,
- X3 représente un atome d'hydrogène,
- R3 et R4 sont tous les deux différents de l'hydrogène et, de préférence, ils représentent tous deux un groupe méthyle.

Selon des variantes préférées, on utilisera, parmi les composé (I) tels que définis ci-dessus :
- les composés de formule : 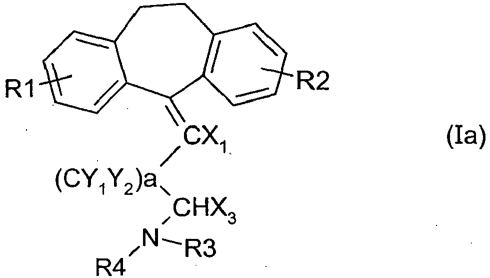 avec R1, R2, X1, a, Y1, Y2, X3, R3 et R4 tels que définis précédemment pour les composés de formule (I),
- les composés de formule : 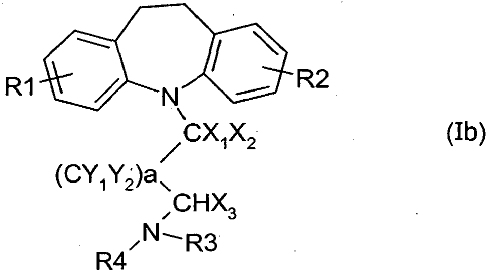 avec R1, R2, X1, X2, a, Y1, Y2, X3, R3 et R4 tels que définis précédemment pour les composés de formule (I),
- les composés de formule : 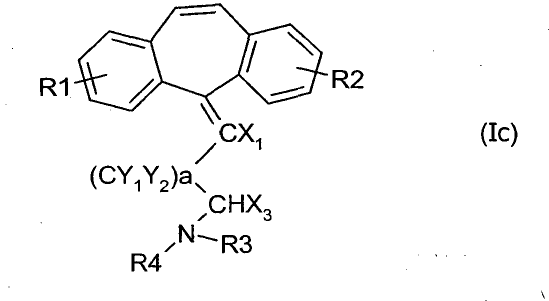 avec R1, R2, X1, a, Y1, Y2, X3, R3 et R4 tels que définis précédemment pour les composés de formule (I),
- les composés de formule : 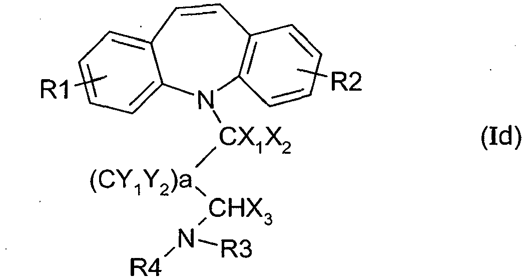 avec R1, R2, X1, X2, a, Y1, Y2, X3, R3 et R4 tels que définis précédemment pour les composés de formule (I),
- les composés de formule : 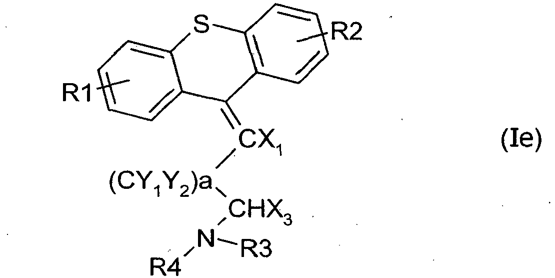 avec R1, R2, X1, a, Y1, Y2, X3, R3 et R4 tels que définis précédemment pour les composés de formule (I),
- les composés de formule : 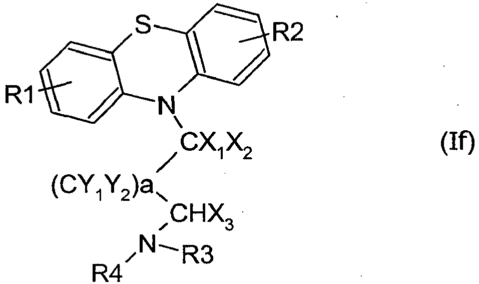 avec R1, R2, X1, X2, a, Y1, Y2, X3, R3 et R4 tels que définis précédemment pour les composés de formule (I),
   éventuellement sous la forme de sels, solvats ou hydrates pharmaceutiquement acceptables.

Selon une variante de l'invention, on utilisera un composé tel que défini ci-dessus, qui permet d'augmenter le taux de sérotonine, dans l'organisme d'un être vivant, et en particulier chez l'homme. Selon une autre variante, le composé utilisé ne présentera pas une telle activité vis-à-vis de la sérotonine. A titre d'exemples de tests permettant de vérifier si un composé est capable ou non d'augmenter le taux de sérotonine dans l'organisme d'un être vivant, on peut citer des tests de réabsorption de la sérotonine dans le cerveau de rat *in vitro* décrits dans les publications suivantes :
- Shank RP, Vaught JL, Pelley KA, Setler PE, McComsey DF, Maryanoff BE, McN-5652: a highly potent inhibitor of serotonin uptake. J Pharm Exp Therap. 1988, 247, 1032-1038
- Hyttel J, Comparative pharmacology of selective serotonin reuptake inhibitors (SSRIs). Nord J Psychiatry. 1993, 47 (suppl 30), 5-12.

En particulier, dans le cadre de l'invention, on entend par «qui permet d'augmenter le taux de sérotonine, dans l'organisme d'un être vivant, et en particulier chez l'homme », un composé qui présente sur au moins l'un de ces tests, ou un test similaire, une IC50 inférieure à 10µM, de préférence inférieure ou égale à 1 µM et préférentiellement inférieure ou égale à 100 nM.

Les sels des composés selon l'invention comprenant notamment un dérivé azoté sont préparés selon des techniques bien connues de l'homme de l'art. Les sels des composés de formule (I) selon la présente invention comprennent ceux avec des acides minéraux ou organiques qui permettent une séparation ou une cristallisation convenable des composés de formule (I), ainsi que des sels pharmaceutiquement acceptables. En tant qu'acide approprié, on peut citer : l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Comme composé sous forme hydratée, on peut citer les semihydrates et monohydrates.

Lorsqu'un composé selon l'invention présente un ou plusieurs carbones asymétriques, les isomères optiques de ce composé font partie intégrante de l'invention. Lorsqu'un composé selon l'invention présente une stéréoisomérie par exemple de type axial-équatorial ou Z-E, l'invention comprend tous les stéréoiosomères de ce composé. La présente invention comprend les composés de formule (I) sous forme d'isomères purs mais également sous forme de mélange d'isomères en proportion quelconque. Les composés (I) sont isolés sous forme d'isomères purs par les techniques classiques de séparation : on pourra utiliser, par exemple des recristallisations fractionnées d'un sel du racémique avec un acide ou une base optiquement active dont le principe est bien connu ou les techniques classiques de chromatographies sur phase chirale ou non chirale.

De façon préférée, l'invention utilise un composé choisi parmi :
- l'amitriptyline (en particulier sous la forme chlorhydrate) de formule : 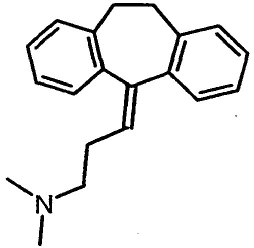
- la cyclobenzaprine : 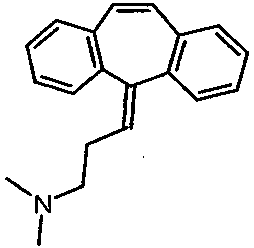
- la désipramine (en particulier sous la forme chlorhydrate) de formule : 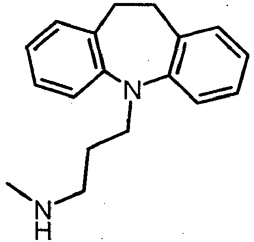
- l'imipramine (en particulier sous la forme chlorhydrate) de formule : 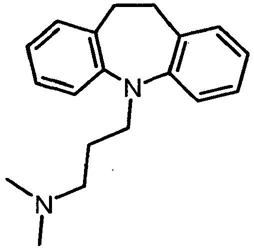
- la trimipramine (en particulier sous la forme maléate) de formule : 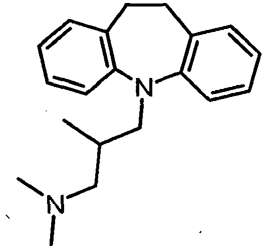
- la méthotriméprazine (en particulier sous la forme maléate) de formule : 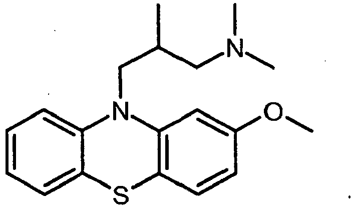
- le chlorprothixene également nommé chloroprothixène (en particulier sous la forme chlorhydrate) de formule : 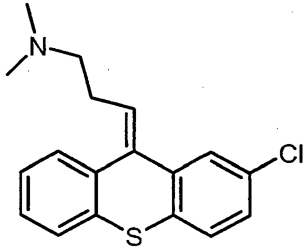
- la triméprazine (en particulier sous la forme tartrate) de formule : 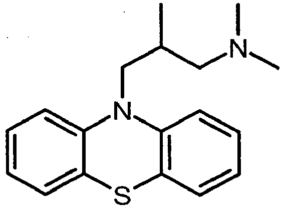
- la clomipramine de formule : 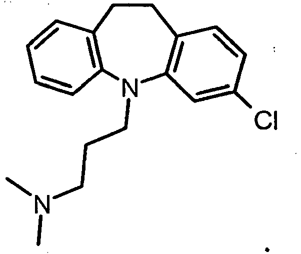
- la norclomipramine de formule : 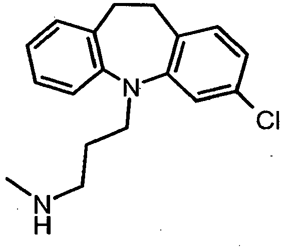
- la nortriptyline de formule : 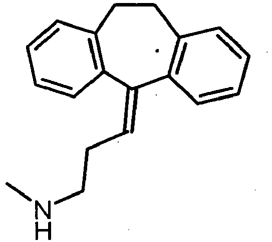
- la maprotiline de formule : 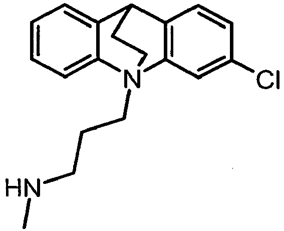
- la chlorpromazine de formule : 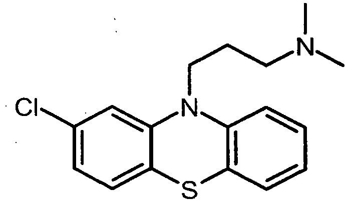
- la promazine de formule : 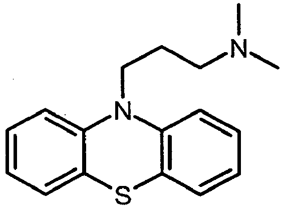
- la prométhazine de formule : 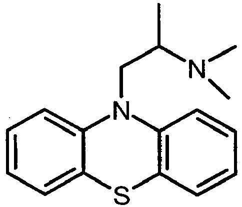
- la perphénazine de formule : 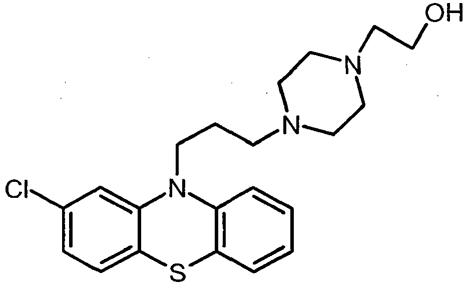
- la trifluperazine de formule : 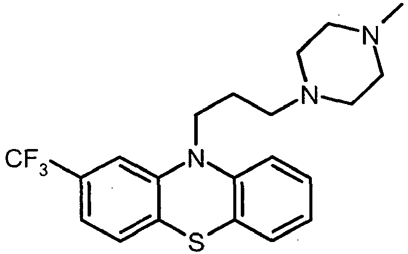
- la thioridazine de formule : 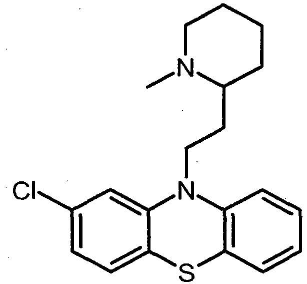
ainsi que leurs sels, solvats ou hydrates pharmaceutiquement acceptables, l'amitriptyline (en particulier sous la forme chlorhydrate), la cyclobenzaprine, la désipramine (en particulier sous la forme chlorhydrate), l'imipramine (en particulier sous la forme chlorhydrate), la trimipramine (en particulier sous la forme maléate), la méthotriméprazine (en particulier sous la forme maléate), le chlorprothixene (en particulier sous la forme chlorhydrate), la triméprazine (en particulier sous la forme tartrate) étant particulièrement préférés.

Pour la synthèse des composés de formule **(Ia)** et **(Ic),** on pourra se référer à S. O., Winthrop; M. A., Davis; G. S., Myers; J. G., Gavin; R., Thomas; R., Barber J. Org. Chem. 1961, 27, 230 et opérer, par exemple selon le schéma ci-après, dans lequel -R représente -(CY1Y2)a-CHX3-NR3R4 :

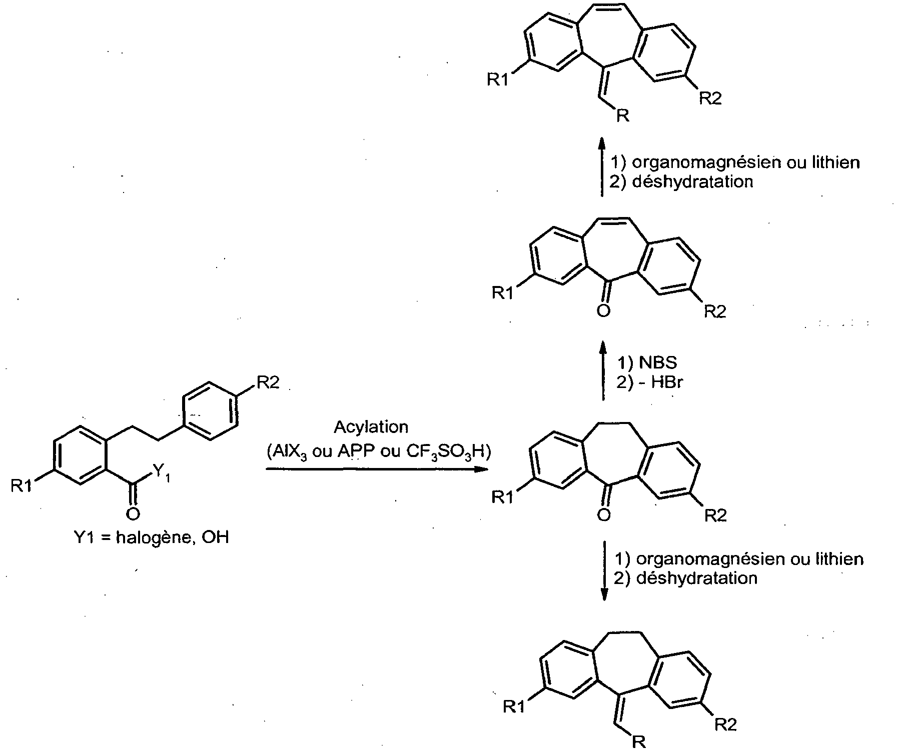

Pour la synthèse des composés de formule **(Ib)** et **(Id)** à noyau dibenzo[*b*,*f*]-azepine, on pourra se référer à R., Huisgen; E. Laschtuvka; F. Bayerlein Chem. Ber. 1960, 93, 392 et P. N., Graig; B. M., Lester; A. J., Saggiomo; C., Kaiser; C. L., Zirkle J. Org. Chem. 1961, 26, 135 et opérer, par exemple selon le schéma ci-après, dans lequel -R' représente -CHX1-(CY1Y2)a-CHX3-NR3R4 :

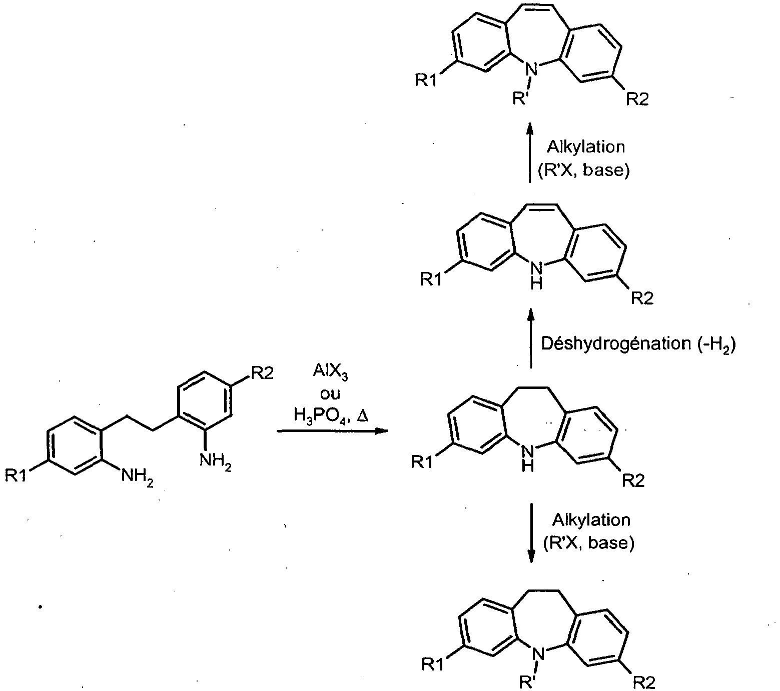

Pour la synthèse des composés de formule (Ie) comportant un noyau thioxanthène, on pourra se référer aux brevets GB 829763, US 2951082 (au nom de Merck) et à GB 834143 (1960 au nom de Am. Cyanamid). Ces composés pourront, par exemple, être synthétisés par alkylation des thioxanthones correspondantes par action d'un organomagnésien ou d'un lithien suivie d'une déshydratation. Selon le brevet US 2951083, on pourra, par exemple, opérer selon le schéma suivant dans lequel -R représente -(CY1Y2)a-CHX3-NR3R4 :

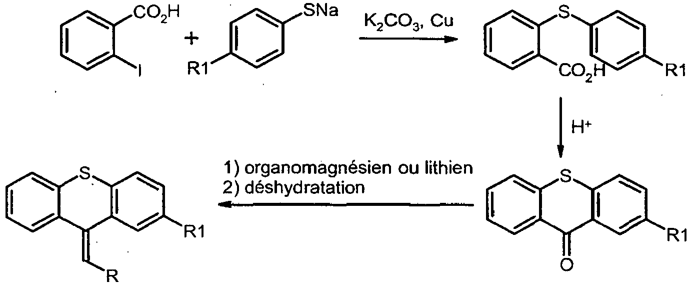

Pour la synthèse des composés de formule (If) comportant un noyau phénothiazine, on pourra se référer à Courvoisier et al. C. R. Soc. Biol. 1977, 151, 1378 et au brevet US2837518 (1958 au nom de Rhone Poulenc). Ces composés sont, par exemple, obtenus par *N*-alkylation des phénothiazines correspondantes selon les procédés décrits dans le brevet US 2837518. Notamment, on procède à l'alkylation en milieu basique par un β-halogéno ester, au passage à l'amide désiré et à la réduction par LiAIH₄. On pourra également se référer à H. L. Yale J. Am. Chem. Soc. 1955, 77, 2270 et A. O. Fitton; R. K. Smalley Pratical Heterocyclic Chemistry; Academic: London 1968: p 127 et opérer, par exemple, selon le schéma ci-après, dans lequel -R' représente -CHX1-(CY1Y2)a-CHX3 -NR3R4:

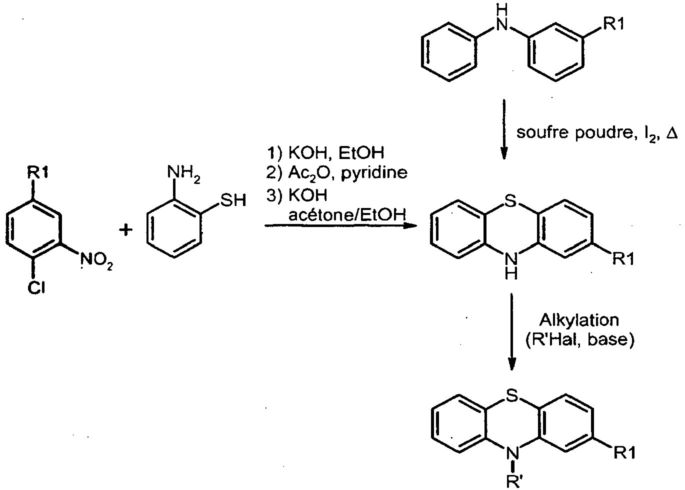

Les pathologies visées dans le cadre de l'invention sont dues à une dégénérescence musculaire du muscle squelettique d'origine génétique, comme les myopathies musculaires. En particulier, l'utilisation des composés capables d'augmenter le taux de sérotonine dans l'organisme est particulièrement adaptée à la préparation de médicaments destinés à traiter les myopathies dégénératives d'origine génétique, et notamment la myopathie de Duchenne.

Certains des composés listés précédemment sont des médicaments commercialisés et pourront être administrés selon des formes pharmaceutiques connues pour d'autres pathologies. Les composés sont conditionnés en mélange avec un ou plusieurs excipients pharmaceutiquement acceptables et les composés peuvent se trouver sous la forme de sels, solvats ou hydrates pharmaceutiquement acceptables. Ces compositions pharmaceutiques sont préparées selon les techniques classiques bien connues de l'homme de l'art. Les excipients pharmaceutiques sont choisis selon la forme pharmaceutique et le mode d'administration souhaité, par exemple, administration orale, sublinguale, sous-cutanée, intramusculaire, intra-veineuse, topique... A titre d'éxemple d'excipients pharmaceutiquement acceptables, on peut citer la silice colloïdale anhydre, glycérol, lactose, magnésium stéarate, amidon de maïs, acide stéarique, talc, hypromellose, polymère de vinylpyrrolidone et vinylacétate, cellulose microcristalline, macrogol 8000, povidone K 30, saccharose, dispersion de fer rouge oxyde dans le dioxyde de titane, titane dioxyde, saccharine sodique, mannitol, sorbitol, anis arôme, menthe arôme, sodium stéarylfumarate...

Les compositions pharmaceutiques pour l'administration orale, sublinguale, sous-cutanée, intramusculaire, intra-veineuse, topique, intratrachéale, intranasale, transdermique, rectale ou intraoculaire, comprenant le composé actif, peuvent être administrées sous formes unitaires d'administration, en mélange avec des supports pharmaceutiques classiques, aux animaux et aux êtres humains pour la prophylaxie ou le traitement des dégénérescences des muscles squelettiques. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

La dose sera adaptée à l'effet prophylactique ou thérapeutique souhaité, à l'age et au poids du patient à traiter. De préférence, la dose journalière sera comprise entre 10 et 500 mg/kg/jour et répartie en 1 à 3 prises. Afin d'obtenir l'effet prophylactique ou thérapeutique désiré, chaque dose unitaire peut contenir de 1 à 500 mg, de composé actif en combinaison avec un support pharmaceutique. Cette dose unitaire peut être administrée 1 à 5 fois par jour de façon à administrer un dosage journalier permettant d'obtenir l'effet souhaité.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols. Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol. Le principe actif peut être formulé également sous forme de microcapsules, éventuellement ave un ou plusieurs supports ou additifs, ou bien avec des matrices telles qu'un polymère ou une cyclodextrine (patch, formes à libération prolongée).

Certains des composés permettant d'augmenter le taux de sérotonine, précédemment cités, font déjà l'objet de médicaments commercialisés et pourront donc être utilisés sous des formulation pharmaceutiques déjà connues pour d'autres indications.

L'imipramine chlorhydrate est, par exemple, commercialisée sous les noms de Antideprin®, Janimine®, Tofranil®.

L'amitriptyline chlorhydrate est, par exemple, commercialisée sous les noms de Elavil®, Tryptanol®, Endep®, Tryptizol®.

La désipramine chlorhydrate est, par exemple, commercialisée sous les noms de Pertrofane® et Nopramine®.

La trimipramine maléate est, par exemple, commercialisée sous les noms de Surmontril® et Rhotrimine®.

Pour plus de détails sur ces compositions, on pourra consulter le VIDAL dans son Edition 2006.

L'activité de ces composés et leur action sur la fonte des muscles squelettiques ont été mises en évidence, par l'inventeur, dans un test effectué chez les nématodes qui jouent le rôle de modèle pour les myopathies dégénératives (Gaud A. et al. Prednisone reduces muscles degeneration in dystrophin-deficient Caenorhabditis elegans, Neuromuscul. Disord. 2004, 14, 365-70). Pour certains composés, l'activité constatée chez les nématodes a été confirmée chez la souris.

Les résultats ci-après sont donnés à titre d'illustration.

### Tests sur le modèle animal Caenorhabditis elegans

### a) Méthode

Les composés ont été testés sur une souche de nématode dépourvue de dystrophine (Gieseler et al., Current Biology, 2000, vol. 10, p1092-1097) et (Ségalat, Neuromuscular Disorders, 2002, Suppl: S105-9). Cette souche est utilisée comme modèle de myopathie dégénérative. Dans cette souche, jusqu'à 30% des cellules musculaires servant à la locomotion (muscles longitudinaux) dégénèrent à l'état adulte (7 jours à 15°C), ce qui se traduit par une paralysie progressive des animaux.

Les composés sont administrés par mélange avec la gélose sur laquelle sont cultivés les animaux. Ils pénètrent par diffusion à travers la cuticule des animaux.

L'effet des composés sur la dégénérescence musculaire est évalué par 1) l'observation du phénotype des animaux (paralysie), et 2) par l'examen cytologique de la. dégénérescence musculaire au moyen de marqueurs musculaires : rodamine-phalloidine et anticorps anti-myosine. Cet examen permet de compter le nombre de cellules musculaires en dégénérescence ou mortes (Gieseler *et al.* , 2000, *supra).*

### b) Résultats

Le tableau suivant donne les résultats obtenus en ce qui concerne la locomotion et la dégénérescence des animaux après 7 jours de culture à 15°C. Chacun des composés diminue la proportion d'animaux paralysés et la dégénérescence musculaire comme le montre le tableau ci-dessous. Par ailleurs, la sérotonine a été testée et diminue également la dégénérescence musculaire. Ce résultat prouve bien que l'augmentation du taux de sérotonine libre dans l'organisme est directement liée à l'effet des composés.

| **Composé testé** | **Concentration (mg/ml)** | **Concentration en mM** | **Pourcentage d'animaux paralysés à J7** | **Diminution de la dégénérescence en %** |
|---|---|---|---|---|
| Amytriptyline chlorhydrate | 0,1 mg/ml | 0,3 mM | 52 | 61 |
| Desipramine chlorhydrate | 0,1 mg/ml | 0,3 mM | 58 | 58 |
| Trimipramine maleate | 0,1 mg/ml | 0,2 mM | 60 | 53 |
| Imipramine chlorhydrate | 0,1 mg/ml | 0,3 mM | 65 | 37 |
| Non traité | - | - | 100 | 0 |

| **Nom de la molécule** | **Concentration efficace (mg/ml)** | **Diminution de la dégénérescence en %** | | |
|---|---|---|---|---|
| Cyclobenzaprine | 0,05 mg/ml | 63 | | |
| Chloroprothixene | 0,02 mg/ml | 58 | | |
| Trimeprazine | 0,1 mg/ml | 65 | | |
| Methotrimeprazine | 0,1 mg/ml | 70 | | |
| Non traité | - | 0 | | |

Note : les concentrations figurant dans ce tableau sont les concentrations dans la gélose de culture. Les concentrations à l'intérieur de l'animal sont inconnues, car la méthode ne permet pas de les mesurer précisément. Les concentrations à l'intérieur de l'animal sont estimées à 100 à 1000 fois inférieures à celles du milieu de la gélose.
c) Concernant l'action sur la cachexie, il a également pu être montré sur un modèle de protéolyse du muscle de C. elegans representatif de la cachexie, et en utilisant le protocole décrit par Zdinak et al., dans Transgene-coded chimeric proteins as reporters of intracellular proteolysis: starvation-induced catabolism of a lacZ fusion protein in muscle cells of Caenorhabditis elegans, J Cell Biochem. 1997 Oct 1;67(1):143-53 que l'imipramine chlorhydrate réduit fortement cette protéolyse (20% de la protéolyse du témoin).

### Tests chez la souris

### Méthode :

Les tests effectués chez la souris sont de plusieurs types : histologiques, fonctionnels sur l'animal entier, et fonctionnels sur muscles disséqués. Les tests sont effectués sur la souris mdx (génotype C57/BL6 mdx5cv), modèle murin de myopathie dégénérative. Ces tests sont notamment détaillés dans les publications suivantes auxquelles on pourra se référer :
De Luca A, et al. Am. J. Pathol. 2005; 166(2), 477-89.
Divet A, etal. Pflugers Arch. 2002 Aug; 444(5), 634-43. Epub 2002 Jul 10.
Rafael JA, et al. Mamm Genome. 2000 Sep ; 11(9), 725-8.

Les composés sont administrés oralement aux souris dans leur nourriture. Les souris sont traitées de l'âge de 2 semaines à 6 semaines. Elles sont testées à 6 semaines.

### Imipramine chlorhydrate (40mg/jour/kg suivant les expériences)

- Réduction de 26% de la dispersion de taille des fibres (marqueur de dégénérescence) par rapport aux animaux non traités.
- Augmentation de 48% du score avec le test d'agrippement.
- Augmentation de 20% de la tension développée par le muscle EDL sur animaux vivants
- Augmentation de 50% de la tension maximale développée par le muscle isolé (protocole de fibres pelées au triton)

### Cyclobenzaprine (3mg/jour/kg)

- Réduction de 52% de la dispersion de taille des fibres (marqueur de dégénérescence) par rapport aux animaux non traités.
- Réduction de 21% de l'indice de dégénérescence du diaphragme (fibres nécrotiques et fibres centronucléées).
- Augmentation de 40% du score le test d'agrippement
- Augmentation de 24% de la force exercée dans test d'agrippement.

Il a également été démontré que la Trimipramine maléate (15 mg/kg/jour) réduit la nécrose du diaphragme (selon le test décrit par Raymackers JM et al. dans Conséquence of parvalbumin deficiency in the mdx mouse: histological, biochemical and mechanical phenotype of a new double mutant. Neuromuscul Disord. 2003 Jun;13(5):376-87), ainsi que le nombre de fibres centronucléées selon le test décrit par Shavlakadze T, et al. dans Targeted expression of insulin-like growth factor-I reduces early myofiber necrosis in dystrophic mdx mice. Mol Ther. 2004 Nov;10(5):829-43), 2 indicateurs de la myopathie : p<5% et p<0,1% respectivement.

## Revendications

1. Composés de formule (I): 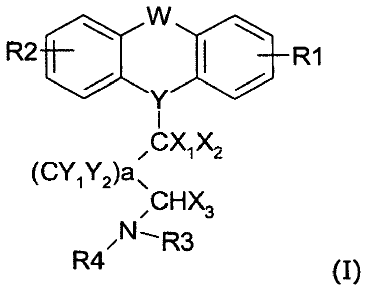 dans laquelle :
- R1 et R2 représentent, chacun indépendamment l'un de l'autre, un atome d'hydrogène ou un atome d'halogène, et en particulier un atome de chlore, ou un groupe trifluorométhyle, un groupe alkyle comprenant de 1 à 5 atomes de carbone, ou un groupe O-alkyle comprenant de 1 à 5 atomes de carbone,
- W représente un atome de soufre ou un enchaînement CH₂-CH₂ ou CH=CH, ou encore un groupe CH lié à Y pour former un pont (CH₂)₂,
- Y représente un atome d'azote ou un atome de carbone, étant entendu que lorsque W et Y sont liés par une chaîne (CH₂)₂, Y est atome de carbone,
- X1 représente un atome d'hydrogène ou un groupe alkyle de 1 à 4 atomes de carbone, ou bien est lié avec R3 pour former un groupe pipéridine,
- X2 est défini comme suit :
lorsque Y représente un atome d'azote ou un atome de carbone lié à W pour former un pont (CH₂)₂, X2 représente un atome d'hydrogène ou un groupe alkyle comprenant de 1 à 4 atomes de carbone,
ou lorsque Y représente un atome de carbone non lié à W pour former un pont (CH₂)₂, X2 forme une liaison covalente entre le carbone auquel il est lié et Y,
de sorte que Y-CX1X2 représente C=CX1,
- a est égal à 0 ou 1,
- Y1 et Y2 représentent, chacun indépendamment l'un de l'autre, un atome d'hydrogène ou un groupe alkyle de 1 à 4 atomes de carbone,
- X3 représente un atome d'hydrogène ou un groupe alkyle de 1 à 4 atomes de carbone, ou X3 est lié avec R3 pour former un groupe pipéridine
- R3 et R4 sont définis comme suit :
o R3 représente un groupe alkyle de 1 à 5 atomes de carbone,
ou bien R3 est lié avec X1 pour former un groupe pipéridine,
ou bien R3 est lié avec X3 pour former un groupe pipéridine,
o R4 représente un atome d'hydrogène ou un groupe alkyle de 1 à 5 atomes de carbone,
∘ ou bien R3 est lié à R4 pour former un groupe pipéridine, pyrrolidine, ou pipérazine dont l'atome d'azote est éventuellement substitué par un groupe alkyle de 1 à 4 atomes de carbone ou par un groupe -(CH₂)_{b}OH avec b qui est égal à 1, 2 ou 3,
éventuellement sous la forme de sels, solvats ou hydrates pharmaceutiquement acceptables, pour leur utilisation dans le traitement ou la prévention des pathologies correspondant à une dégénérescence musculaire des muscles squelettiques choisies parmi les myopathies musculaires d'origine génétique.

2. Utilisation d'un composé de formule (I): 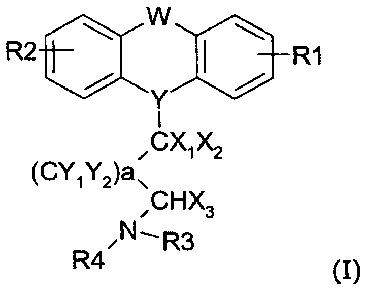 dans laquelle :
- R1 et R2 représentent, chacun indépendamment l'un de l'autre, un atome d'hydrogène ou un atome d'halogène, et en particulier un atome de chlore, ou un groupe trifluorométhyle, un groupe alkyle comprenant de 1 à 5 atomes de carbone, ou un groupe O-alkyle comprenant de 1 à 5 atomes de carbone,
- W représente un atome de soufre ou un enchaînement CH₂-CH₂ ou CH=CH, ou encore un groupe CH lié à Y pour former un pont (CH₂)₂,
- Y représente un atome d'azote ou un atome de carbone, étant entendu que lorsque W et Y sont liés par une chaîne (CH₂)₂, Y est atome de carbone,
- X1 représente un atome d'hydrogène ou un groupe alkyle de 1 à 4 atomes de carbone, ou bien est lié avec R3 pour former un groupe pipéridine,
- X2 est défini comme suit :
lorsque Y représente un atome d'azote ou un atome de carbone lié à W pour former un pont (CH₂)₂, X2 représente un atome d'hydrogène ou un groupe alkyle comprenant de 1 à 4 atomes de carbone,
ou lorsque Y représente un atome de carbone non lié à W pour former un pont (CH₂)₂, X2 forme une liaison covalente entre le carbone auquel il est lié et Y, de sorte que Y-CX1X2 représente C=CX1,
- a est égal à 0 ou 1,
- Y1 et Y2 représentent, chacun indépendamment l'un de l'autre, un atome d'hydrogène ou un groupe alkyle de 1 à 4 atomes de carbone,
- X3 représente un atome d'hydrogène ou un groupe alkyle de 1 à 4 atomes de carbone, ou X3 est lié avec R3 pour former un groupe pipéridine
- R3 et R4 sont définis comme suit :
o R3 représente un groupe alkyle de 1 à 5 atomes de carbone,
ou bien R3 est lié avec X1 pour former un groupe pipéridine,
ou bien R3 est lié avec X3 pour former un groupe pipéridine,
o R4 représente un atome d'hydrogène ou un groupe alkyle de 1 à 5 atomes de carbone,
∘ ou bien R3 est lié à R4 pour former un groupe pipéridine, pyrrolidine, ou pipérazine dont l'atome d'azote est éventuellement substitué par un groupe alkyle de 1 à 4 atomes de carbone ou par un groupe -(CH2)_{b}OH avec b qui est égal à 1, 2 ou 3,
éventuellement sous la forme de sels, solvats ou hydrates pharmaceutiquement acceptables, pour la fabrication d'un médicament destiné à traiter ou à prévenir des pathologies correspondant à une dégénérescence musculaire des muscles squelettiques choisies parmi les myopathies musculaires d'origine génétique.

3. Composés pour utilisation selon la revendication 1 ou utilisation selon la revendication 2 caractérisé(s) en ce que :
- R1 et R2 représentent, chacun indépendamment l'un de l'autre, un atome d'hydrogène ou un atome d'halogène, et en particulier un atome de chlore, ou un groupe méthyle, trifluorométhyle ou méthoxy,
- W représente un atome de soufre ou un enchaînement CH₂-CH₂ ou CH=CH, ou encore un groupe CH lié à Y pour former un pont (CH₂)₂,
- Y représente un atome d'azote ou un atome de carbone, étant entendu que lorsque W et Y sont liés par une chaîne (CH₂)₂, Y est atome de carbone,
- X1 représente un atome d'hydrogène,
- X2 est défini comme suit :
lorsque Y représente un atome d'azote ou un atome de carbone lié à W pour former un pont (CH₂)₂, X2 représente un atome d'hydrogène,
ou lorsque Y représente un atome de carbone non lié à W pour former un pont (CH₂)₂, X2 forme une liaison covalente entre le carbone auquel il est lié et Y, de sorte que Y-CX1X2 représente C=CX1,
- a est égal à 0 ou 1,
- Y1 représente un atome d'hydrogène et Y2 représente un atome d'hydrogène ou un groupe méthyle,
- X3 représente un atome d'hydrogène ou un groupe méthyle, ou X3 est lié avec R3 pour former un groupe pipéridine,
- R3 et R4 sont définis comme suit :
o R3 représente un groupe méthyle,
ou bien R3 est lié avec X3 pour former un groupe pipéridine,
o R4 représente un atome d'hydrogène ou un groupe méthyle,
∘ ou bien R3 est lié à R4 pour former un groupe pipéridine, ou pipérazine dont l'atome d'azote est éventuellement substitué par un groupe méthyle ou par un groupe -(CH2)_{b}OH avec b qui est égal à 2.

4. Composés pour utilisation selon la revendication 1 ou 3 ou utilisation selon la revendication 2 ou 3 caractérisé(s) en ce que R1 et R2 se trouvent, chacun indépendamment l'un de l'autre, en position 2 ou 3 du groupe phényle auquel ils sont liés.

5. Composés pour utilisation selon la revendication 1,3 ou 4, ou utilisation selon l'une des revendications 2 à 4 caractérisé(s) en ce que a est égal à 1.

6. Composés pour utilisation selon l'une des revendications 1 et 3 à 5, ou utilisation selon l'une des revendications 2 à 5 caractérisé(s) en ce que X3 représente un atome d'hydrogène.

7. Composés pour utilisation selon l'une des revendications 1 et 3 à 6, ou utilisation selon l'une des revendications 2 à 6 **caractérisés en ce que** R3 et R4 sont tous les deux différents de l'hydrogène et, de préférence, ils représentent tous deux un groupe méthyle.

8. Composé pour utilisation selon la revendication 1 ou 3, ou utilisation selon la revendication 2 ou 3 caractérisé(s) en ce que le composé est choisi parmi l'amitriptyline (en particulier sous la forme chlorhydrate) de formule : 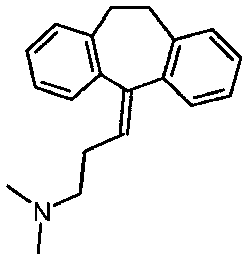
- la cyclobenzaprine : 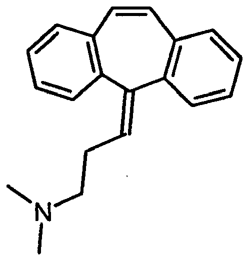
- la désipramine (en particulier sous la forme chlorhydrate) de formule : 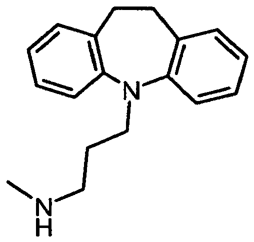
- l'imipramine (en particulier sous la forme chlorhydrate) de formule : 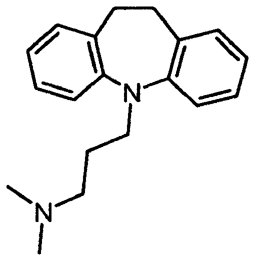
- la trimipramine (en particulier sous la forme maléate) de formule : 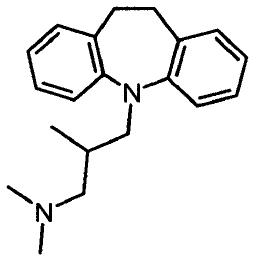
- la méthotriméprazine (en particulier sous la forme maléate) de formule : 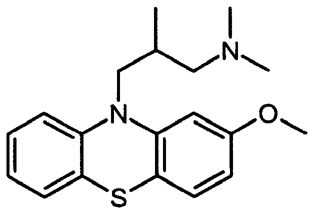
- le chlorprothixene (en particulier sous la forme chlorhydrate) de formule : 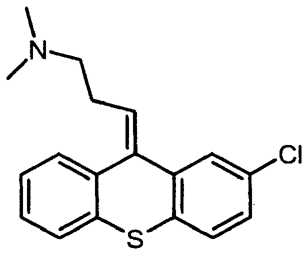
- la triméprazine (en particulier sous la forme tartrate) de formule : 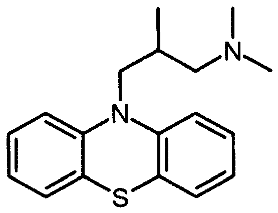
- la clomipramine de formule : 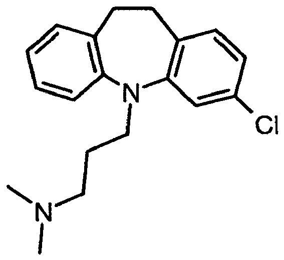
- la norclomipramine de formule : 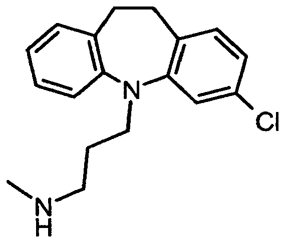
- la nortriptyline de formule : 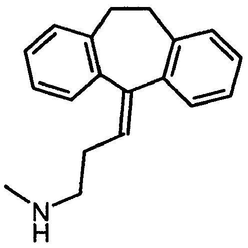
- la maprotiline de formule : 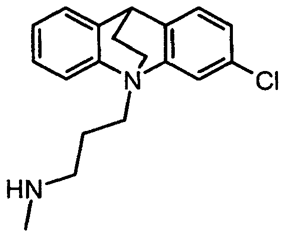
- la chlorpromazine de formule : 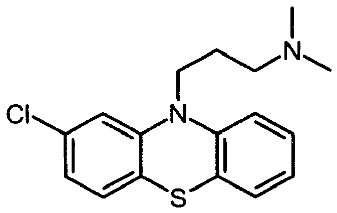
- la promazine de formule : 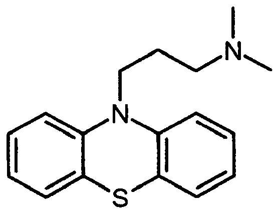
- la prométhazine de formule : 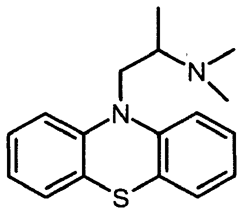
- la perphénazine de formule : 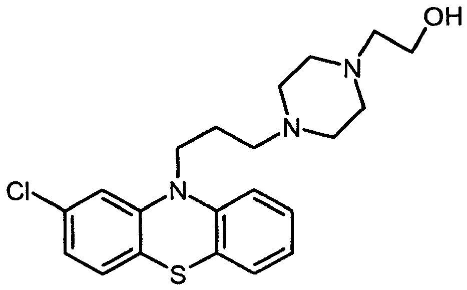
- la trifluperazine de formule : 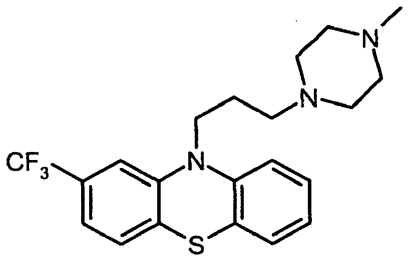
- la thioridazine de formule : 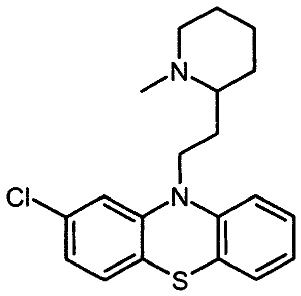 ou un de leurs sels, solvats ou hydrates pharmaceutiquement acceptables.

9. Composé pour utilisation selon l'une des revendications 1 et 3 à 8 ou utilisation selon l'une des revendications 2 à 8 **caractérisé en ce que** le composé est choisi parmi l'amitriptyline en particulier sous la forme chlorhydrate, la cyclobenzaprine, la désipramine en particulier sous la forme chlorhydrate, l'imipramine en particulier sous la forme chlorhydrate, la trimipramine en particulier sous la forme maléate, la méthotriméprazine en particulier sous la forme maléate, le chlorprothixene en particulier sous la forme chlorhydrate, ou la triméprazine en particulier sous la forme tartrate.

10. Composés pour utilisation selon l'une des revendications 1 et 3 à 9 pour le traitement ou la prévention de la myopathie de Duchenne.

11. Utilisation selon l'une des revendications 2 à 9 **caractérisée en ce que** le médicament est destiné au traitement ou à la prévention de la myopathie de Duchenne.

12. Composé pour utilisation selon l'une des revendications 1 et 3 à 10 ou utilisation selon l'une des revendications 2 à 9 et 11 **caractérisé en ce que** ledit composé permet d'augmenter le taux de sérotonine, dans l'organisme d'un être vivant, et en particulier chez l'homme.

13. Composés pour utilisation selon l'une des revendications 1 et 3 à 10 ou utilisation selon l'une des revendications 2 à 9 et 11 **caractérisé en ce que** ledit composé ne permet pas d'augmenter le taux de sérotonine, dans l'organisme d'un être vivant, et en particulier chez l'homme.

## Patentansprüche

1. Verbindungen der Formel (I): 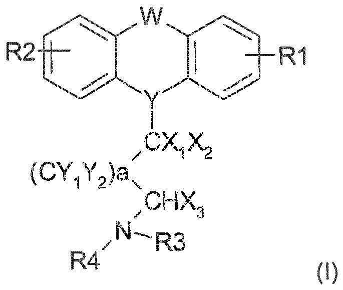 worin:
- R1 und R2, jeweils unabhängig voneinander, ein Wasserstoffatom oder ein Halogenatom, und insbesondere ein Chloratom, oder eine Trifluormethylgruppe, eine Alkylgruppe mit 1 bis 5 Kohlenstoffatomen oder eine O-Alkylgruppe mit 1 bis 5 Kohlenstoffatomen bedeuten,
- W ein Schwefelatom oder eine CH₂-CH₂- oder CH=CH-Verkettung oder aber eine an Y gebundene CH-Gruppe zur Bildung einer (CH₂)₂-Brücke bedeutet,
- Y ein Stickstoffatom oder ein Kohlenstoffatom bedeutet, mit der Maßgabe, dass wenn W und Y durch eine (CH₂)₂-Kette verbunden sind, Y Kohlenstoffatom ist,
- X1 ein Wasserstoffatom oder eine Alkylgruppe mit 1 bis 4 Kohlenstoffatomen bedeutet oder mit R3 verbunden ist, um eine Piperidingruppe zu bilden,
- X2 wie folgt definiert ist:
wenn Y ein Stickstoffatom oder ein an W gebundenes Kohlenstoffatom zur Bildung einer (CH₂)₂-Brücke bedeutet, X2 ein Wasserstoffatom oder eine Alkylgruppe mit 1 bis 4 Kohlenstoffatomen bedeutet,
oder wenn Y ein nicht an W gebundenes Kohlenstoffatom zur Bildung einer (CH₂)₂-Brücke bedeutet, X2 eine kovalente Bindung zwischen dem Kohlenstoff, an den es gebunden ist, und Y bildet, so dass Y-CX1X2 C=CX1 bedeutet,
- a gleich 0 oder 1 ist,
- Y1 und Y2, jeweils unabhängig voneinander, ein Wasserstoffatom oder eine Alkylgruppe mit 1 bis 4 Kohlenstoffatomen bedeuten,
- X3 ein Wasserstoffatom oder eine Alkylgruppe mit 1 bis 4 Kohlenstoffatomen bedeutet oder X3 mit R3 verbunden ist, um eine Piperidingruppe zu bilden,
- R3 und R4 wie folgt definiert sind:
∘ R3 bedeutet eine Alkylgruppe mit 1 bis 5 Kohlenstoffatomen,
oder R3 ist mit X1 verbunden, um eine Piperidingruppe zu bilden,
oder R3 ist mit X3 verbunden, um eine Piperidingruppe zu bilden,
∘ R4 bedeutet ein Wasserstoffatom oder eine Alkylgruppe mit 1 bis 5 Kohlenstoffatomen,
∘ oder R3 ist an R4 gebunden, um eine Piperidin-, Pyrrolidin- oder Piperazingruppe zu bilden, deren Stickstoffatom eventuell durch eine Alkylgruppe mit 1 bis 4 Kohlenstoffatomen oder durch eine -(CH₂)_{b}OH-Gruppe, wobei b gleich 1, 2 oder 3 ist, substituiert ist,
eventuell in Form von pharmazeutisch akzeptablen Salzen, Solvaten oder Hydraten, für ihre Verwendung bei der Behandlung oder der Prävention von einer Muskeldegeneration der Skelettmuskulatur entsprechenden Pathologien, die aus den Muskelmyopathien genetischen Ursprungs ausgewählt sind.

2. Verwendung einer Verbindung der Formel (I): 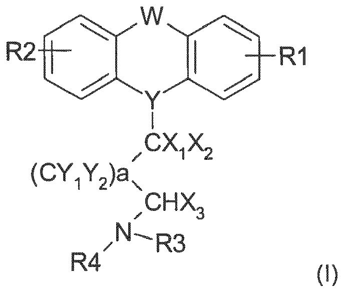 worin:
- R1 und R2, jeweils unabhängig voneinander, ein Wasserstoffatom oder ein Halogenatom, und insbesondere ein Chloratom, oder eine Trifluormethylgruppe, eine Alkylgruppe mit 1 bis 5 Kohlenstoffatomen oder eine O-Alkylgruppe mit 1 bis 5 Kohlenstoffatomen bedeuten,
- W ein Schwefelatom oder eine CH₂-CH₂- oder CH=CH-Verkettung oder aber eine an Y gebundene CH-Gruppe zur Bildung einer (CH₂)₂-Brücke bedeutet,
- Y ein Stickstoffatom oder ein Kohlenstoffatom bedeutet, mit der Maßgabe, dass wenn W und Y durch eine (CH₂)₂-Kette verbunden sind, Y Kohlenstoffatom ist,
- X1 ein Wasserstoffatom oder eine Alkylgruppe mit 1 bis 4 Kohlenstoffatomen bedeutet oder mit R3 verbunden ist, um eine Piperidingruppe zu bilden,
- X2 wie folgt definiert ist:
wenn Y ein Stickstoffatom oder ein an W gebundenes Kohlenstoffatom zur Bildung einer (CH₂)₂-Brücke bedeutet, X2 ein Wasserstoffatom oder eine Alkylgruppe mit 1 bis 4 Kohlenstoffatomen bedeutet,
oder wenn Y ein nicht an W gebundenes Kohlenstoffatom zur Bildung einer (CH₂)₂-Brücke bedeutet, X2 eine kovalente Bindung zwischen dem Kohlenstoff, an den es gebunden ist, und Y bildet, so dass Y-CX1X2 C=CX1 bedeutet,
- a gleich 0 oder 1 ist,
- Y1 und Y2, jeweils unabhängig voneinander, ein Wasserstoffatom oder eine Alkylgruppe mit 1 bis 4 Kohlenstoffatomen bedeuten,
- X3 ein Wasserstoffatom oder eine Alkylgruppe mit 1 bis 4 Kohlenstoffatomen bedeutet oder X3 mit R3 verbunden ist, um eine Piperidingruppe zu bilden,
- R3 und R4 wie folgt definiert sind:
∘ R3 bedeutet eine Alkylgruppe mit 1 bis 5 Kohlenstoffatomen,
oder R3 ist mit X1 verbunden, um eine Piperidingruppe zu bilden,
oder R3 ist mit X3 verbunden, um eine Piperidingruppe zu bilden,
∘ R4 bedeutet ein Wasserstoffatom oder eine Alkylgruppe mit 1 bis 5 Kohlenstoffatomen,
∘ oder R3 ist an R4 gebunden, um eine Piperidin-, Pyrrolidin- oder Piperazingruppe zu bilden, deren Stickstoffatom eventuell durch eine Alkylgruppe mit 1 bis 4 Kohlenstoffatomen oder durch eine -(CH₂)_{b}OH-Gruppe, wobei b gleich 1, 2 oder 3 ist, substituiert ist,
eventuell in Form von pharmazeutisch akzeptablen Salzen, Solvaten oder Hydraten, für die Herstellung eines Medikaments, das für die Behandlung oder die Prävention von einer Muskeldegeneration der Skelettmuskulatur entsprechenden Pathologien, die aus den Muskelmyopathien genetischen Ursprungs ausgewählt sind, bestimmt ist.

3. Verbindungen zur Verwendung nach Anspruch 1 oder Verwendung nach Anspruch 2, **dadurch gekennzeichnet, dass**:
- R1 und R2, jeweils unabhängig voneinander, ein Wasserstoffatom oder ein Halogenatom, und insbesondere ein Chloratom, oder eine Methyl-, Trifluormethyl- oder Methoxygruppe bedeuten,
- W ein Schwefelatom oder eine CH₂-CH₂- oder CH=CH-Verkettung oder aber eine an Y gebundene CH-Gruppe zur Bildung einer (CH₂)₂-Brücke bedeutet,
- Y ein Stickstoffatom oder ein Kohlenstoffatom bedeutet, mit der Maßgabe, dass wenn W und Y durch eine (CH₂)₂-Kette verbunden sind, Y Kohlenstoffatom ist,
- X1 ein Wasserstoffatom bedeutet,
- X2 wie folgt definiert ist:
wenn Y ein Stickstoffatom oder ein an W gebundenes Kohlenstoffatom zur Bildung einer (CH₂)₂-Brücke bedeutet, X2 ein Wasserstoffatom bedeutet,
oder wenn Y ein nicht an W gebundenes Kohlenstoffatom zur Bildung einer (CH₂)₂-Brücke bedeutet, X2 eine kovalente Bindung zwischen dem Kohlenstoff, an den es gebunden ist, und Y bildet, so dass Y-CX1X2 C=CX1 bedeutet,
- a gleich 0 oder 1 ist,
- Y1 ein Wasserstoffatom bedeutet und Y2 ein Wasserstoffatom oder eine Methylgruppe bedeutet,
- X3 ein Wasserstoffatom oder eine Methylgruppe bedeutet oder X3 mit R3 verbunden ist, um eine Piperidingruppe zu bilden,
- R3 und R4 wie folgt definiert sind:
∘ R3 bedeutet eine Methylgruppe,
oder R3 ist mit X3 verbunden, um eine Piperidingruppe zu bilden,
∘ R4 bedeutet ein Wasserstoffatom oder eine Methylgruppe,
∘ oder R3 ist an R4 gebunden, um eine Piperidin- oder Piperazingruppe zu bilden, deren Stickstoffatom eventuell durch eine Methylgruppe oder durch eine -(CH₂)_{b}OH-Gruppe, wobei b gleich 2 ist, substituiert ist.

4. Verbindungen zur Verwendung nach Anspruch 1 oder 3 oder Verwendung nach Anspruch 2 oder 3, **dadurch gekennzeichnet, dass** R1 und R2 sich jeweils unabhängig voneinander in 2- oder 3-Stellung der Phenylgruppe, an die sie gebunden sind, befinden.

5. Verbindungen zur Verwendung nach Anspruch 1, 3 oder 4 oder Verwendung nach einem der Ansprüche 2 bis 4, **dadurch gekennzeichnet, dass** a gleich 1 ist.

6. Verbindungen zur Verwendung nach einem der Ansprüche 1 und 3 bis 5 oder Verwendung nach einem der Ansprüche 2 bis 5, **dadurch gekennzeichnet, dass** X3 ein Wasserstoffatom bedeutet.

7. Verbindungen zur Verwendung nach einem der Ansprüche 1 und 3 bis 6 oder Verwendung nach einem der Ansprüche 2 bis 6, **dadurch gekennzeichnet, dass** R3 und R4 alle beide von Wasserstoff verschieden sind und vorzugsweise alle beide eine Methylgruppe bedeuten.

8. Verbindung zur Verwendung nach Anspruch 1 oder 3 oder Verwendung nach 2 oder 3, **dadurch gekennzeichnet, dass** die Verbindung ausgewählt ist aus Amitriptylin (insbesondere in Chlorhydratform) der Formel: 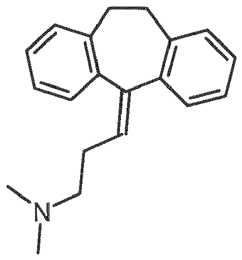
- Cyclobenzaprin: 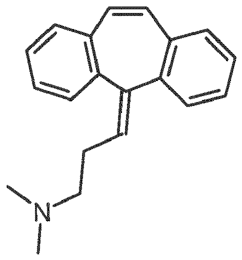
- Desipramin (insbesondere in Chlorhydratform) der Formel: 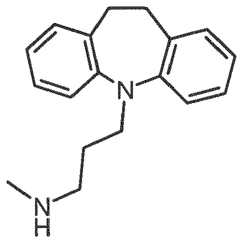
- Imipramin (insbesondere in Chlorhydratform) der Formel: 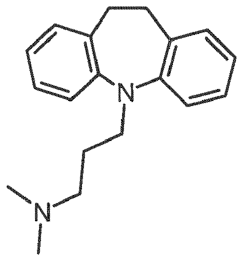
- Trimipramin (insbesondere in Maleatform) der Formel: 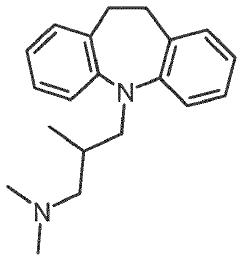
- Methotrimeprazin (insbesondere in Maleatform) der Formel: 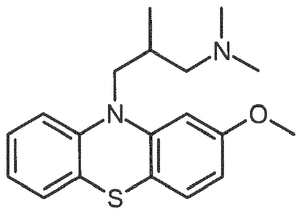
- Chlorprothixen (insbesondere in Chlorhydratform) der Formel: 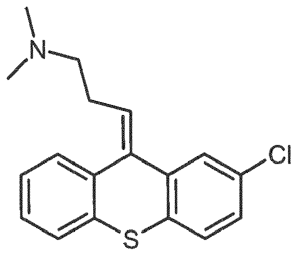
- Trimeprazin (insbesondere in Tartratform) der Formel: 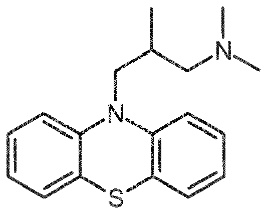
- Clomipramin der Formel: 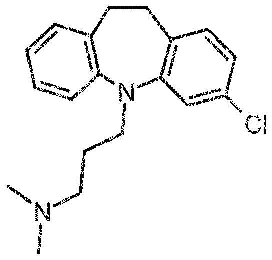
- Norclomipramin der Formel: 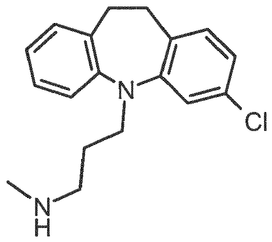
- Nortriptylin der Formel: 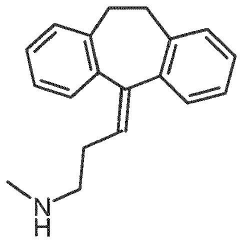
- Maprotilin der Formel: 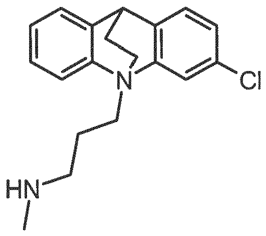
- Chlorpromazin der Formel: 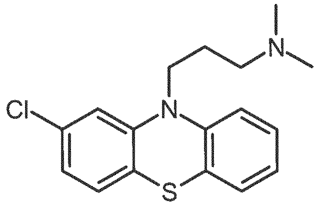
- Promazin der Formel: 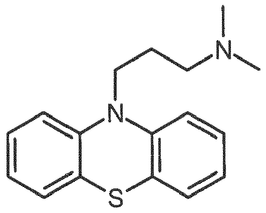
- Promethazin der Formel: 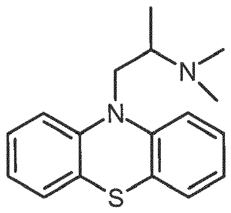
- Perphenazin der Formel: 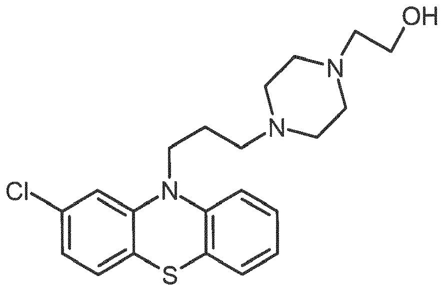
- Trifluperazin der Formel: 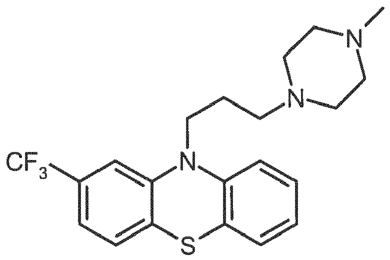
- Thioridazin der Formel: 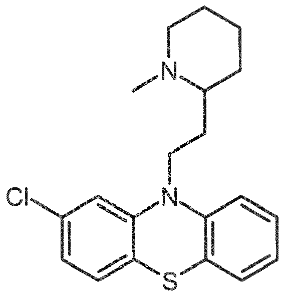 oder eines ihrer pharmazeutisch akzeptablen Salze, Solvate oder Hydrate.

9. Verbindung zur Verwendung nach einem der Ansprüche 1 und 3 bis 8 oder Verwendung nach einem der Ansprüche 2 bis 8, **dadurch gekennzeichnet, dass** die Verbindung ausgewählt ist aus Amitriptylin insbesondere in Chlorhydratform, Cyclobenzaprin, Desipramin insbesondere in Chlorhydratform, Imipramin insbesondere in Chlorhydratform, Trimipramin insbesondere in Maleatform, Methotrimeprazin insbesondere in Maleatform, Chlorprothixen insbesondere in Chlorhydratform, oder Trimeprazin insbesondere in Tartratform.

10. Verbindungen zur Verwendung nach einem der Ansprüche 1 und 3 bis 9 für die Behandlung oder die Prävention der Muskeldystrophie vom Typ Duchenne.

11. Verwendung nach einem der Ansprüche 2 bis 9, **dadurch gekennzeichnet, dass** das Medikament für die Behandlung oder die Prävention der Muskeldystrophie vom Typ Duchenne bestimmt ist.

12. Verbindung zur Verwendung nach einem der Ansprüche 1 und 3 bis 10 oder Verwendung nach einem der Ansprüche 2 bis 9 und 11, **dadurch gekennzeichnet, dass** die Verbindung ermöglicht, den Serotoninspiegel im Organismus eines Lebewesens und insbesondere beim Menschen zu erhöhen.

13. Verbindungen zur Verwendung nach einem der Ansprüche 1 und 3 bis 10 oder Verwendung nach einem der Ansprüche 2 bis 9 und 11, **dadurch gekennzeichnet, dass** die Verbindung nicht ermöglicht, den Serotoninspiegel im Organismus eines Lebewesens und insbesondere beim Menschen zu erhöhen.

## Claims

1. Compounds of formula (I): 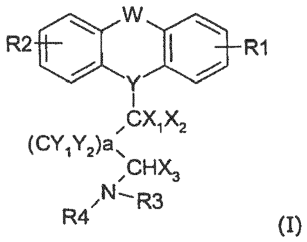 where:
- R1 and R2 are each independently a hydrogen atom or halogen atom, in particular a chlorine atom, or trifluoromethyl group, alkyl group having 1 to 5 carbon atoms, or O-alkyl group having 1 to 5 carbon atoms;
- W is a sulfur atom or CH₂-CH₂ or CH=CH sequence, or a CH group attached to Y to form a bridge (CH₂)₂;
- Y is a nitrogen atom or carbon atom on the understanding that when W and Y are attached by a (CH₂)₂ chain, Y is a carbon atom;
- X1 is a hydrogen atom or alkyl group having 1 to 4 carbon atoms or else it is attached to R3 to form a piperidine group.
- X2 is defined as follows:
when Y is a nitrogen atom or carbon atom attached to W to form a (CH₂)₂ bridge, X2 is a hydrogen atom or an alkyl group having 1 to 4 carbon atoms,
or when Y is a carbon atom not attached to W to form a (CH₂)₂ bridge, X2 forms a covalent bond between the carbon to which it is attached and Y, so that Y-CX1X2 represents C=CX1,
- a equals 0 or 1;
- Y1 and Y2 are each independently a hydrogen atom or an alkyl group having 1 to 4 carbon atoms;
- X3 is a hydrogen atom or an alkyl group having 1 to 4 carbon atoms, or X3 is attached to R3 to form a piperidine group
- R3 and R4 are defined as follows:
∘ R3 is an alkyl group having 1 to 5 carbon atoms,
or else R3 is attached to X1 to form a piperidine group,
or else R3 is attached to X3 to form a piperidine group;
∘ R4 is a hydrogen atom or an alkyl group having 1 to 5 carbon atoms;
∘ or else R3 is attached to R4 to form a piperidine, pyrrolidine or piperazine group the nitrogen atom of which is optionally substituted by an alkyl group having 1 to 4 carbon atoms or by a -(CH2)_{b}OH group where b equals 1, 2 or 3,
optionally in the form of pharmaceutically acceptable salts, solvates or hydrates for use thereof in the treatment or prevention of pathologies corresponding to muscular degeneration of skeletal muscles selected from among muscular myopathies of genetic origin.

2. The use of a compound of formula (I): 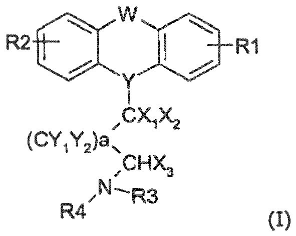 where:
- R1 and R2 are each independently a hydrogen atom or halogen atom, in particular a chlorine atom, or trifluoromethyl group, alkyl group having 1 to 5 carbon atoms, or O-alkyl group having 1 to 5 carbon atoms;
- W is a sulfur atom or CH₂-CH₂ or CH=CH sequence, or a CH group attached to Y to form a bridge (CH₂)₂;
- Y is a nitrogen atom or carbon atom on the understanding that when W and Y are attached by a (CH₂)₂ chain, Y is a carbon atom;
- X1 is a hydrogen atom or alkyl group having 1 to 4 carbon atoms, or else it is attached to R3 to form a piperidine group;
- X2 is defined as follows:
when Y is a nitrogen atom or carbon atom attached to W to form a (CH₂)₂ bridge, X2 is a hydrogen atom or an alkyl group having 1 to 4 carbon atoms,
or when Y is a carbon atom not attached to W to form a (CH₂)₂ bridge, X2 forms a covalent bond between the carbon to which it is attached and Y, so that Y-CX1X2 represents C=CX1,
- a equals 0 or 1;
- Y1 and Y2 are each independently a hydrogen atom or an alkyl group having 1 to 4 carbon atoms;
- X3 is a hydrogen atom or an alkyl group having 1 to 4 carbon atoms, or X3 is attached to R3 to form a piperidine group
- R3 and R4 are defined as follows:
∘ R3 is an alkyl group having 1 to 5 carbon atoms,
or else R3 is attached to X1 to form a piperidine group,
or else R3 is attached to X3 to form a piperidine group,
∘ R4 is a hydrogen atom or an alkyl group having 1 to 5 carbon atoms,
∘ or else R3 is attached to R4 to form a piperidine, pyrrolidine or piperazine group the nitrogen atom of which is optionally substituted by an alkyl group having 1 to 4 carbon atoms or by a -(CH2)_{b}OH group where b equals 1, 2 or 3,
optionally in the form of pharmaceutically acceptable salts, solvates or hydrates for producing a drug for the treatment or prevention of pathologies corresponding to muscular degeneration of the skeletal muscles selected from among muscular myopathies of genetic origin.

3. Compounds for use according to claim 1, or use according to claim 2 **characterized in that**:
- R1 and R2 are each independently a hydrogen atom or halogen atom, in particular a chlorine atom, or a methyl, trifluoromethyl or methoxy group;
- W is a sulfur atom or CH₂-CH₂ or CH=CH sequence, or a CH group attached to Y to form a bridge (CH₂)₂;
- Y is a nitrogen atom or carbon atom on the understanding that when W and Y are attached by a (CH₂)₂ chain, Y is a carbon atom;
- X1 is a hydrogen atom;
- X2 is defined as follows:
when Y is a nitrogen atom or carbon atom attached to W to form a (CH₂)₂ bridge, X2 is a hydrogen atom,
or when Y is a carbon atom not attached to W to form a (CH₂)₂ bridge, X2 forms a covalent bond between the carbon to which it is attached and Y, so that Y-CX1X2 represents C=CX1,
- a equals 0 or 1;
- Y1 is a hydrogen atom and Y2 is a hydrogen atom or a methyl group;
- X3 is a hydrogen atom or a methyl group, or X3 is attached to R3 to form a piperidine group;
- R3 and R4 are defined as follows:
∘ R3 is a methyl group,
or else R3 is attached to X3 to form a piperidine group;
∘ R4 is a hydrogen atom or methyl group;
∘ or else R3 is attached to R4 to form a piperidine or piperazine group the nitrogen atom of which is optionally substituted by a methyl group or by a -(CH2)_{b}OH group where b equals 2.

4. Compounds for use according to claim 1 or 3, or use according to claim 2 or 3, **characterized in that** R1 and R2 each independently lie at position 2 or 3 of the phenyl group to which they are attached.

5. Compounds for use according to claim 1, 3 or 4, or use according to one of claims 2 to 4, **characterized in that** a equals 1.

6. Compounds for use according to one of claims 1 and 3 to 5, or use according to one of claims 2 to 5, **characterized in that** X3 is a hydrogen atom.

7. Compounds for use according to one of claims 1 and 3 to 6, or use according to one of claims 2 to 6, **characterized in that** R3 and R4 both differ from hydrogen and are preferably both a methyl group.

8. A compound for use according to claim 1 or 3, or use according to claim 2 or 3, **characterized in that** the compound is selected from among:
- amitriptyline (in particular the hydrochloride form) of formula: 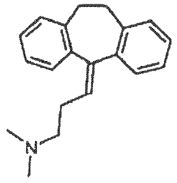
- cyclobenzaprine: 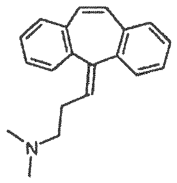
- desipramine (in particular the hydrochloride form) of formula: 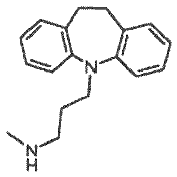
- imipramine (in particular the hydrochloride form) of formula: 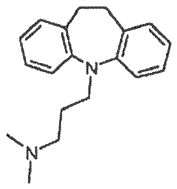
- trimipramine (in particular the maleate form) of formula: 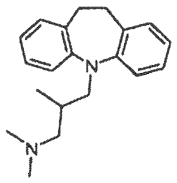
- methotrimeprazine (in particular the maleate form) of formula: 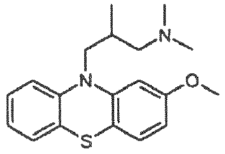
- chlorprothixene (in particular the hydrochloride form) of formula: 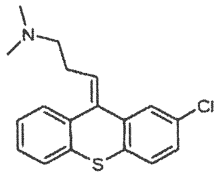
- trimeprazine (in particular the tartrate form) of formula: 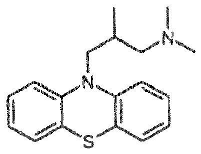
- clomipramine of formula: 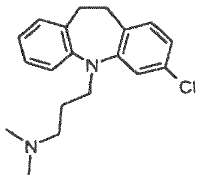
- norclomipramine of formula: 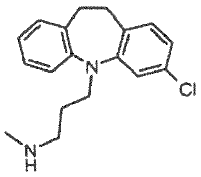
- nortriptyline of formula: 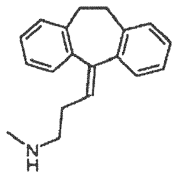
- maprotiline of formula: 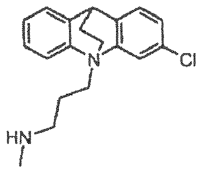
- chlorpromazine of formula: 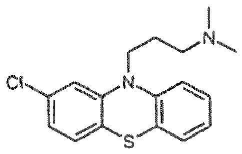
- promazine of formula: 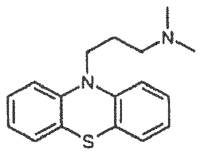
- promethazine of formula: 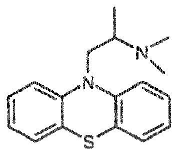
- perphenazine of formula: 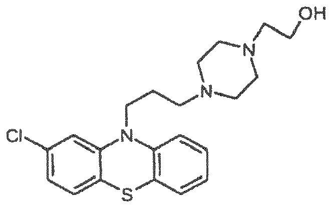
- trifluperazine of formula: 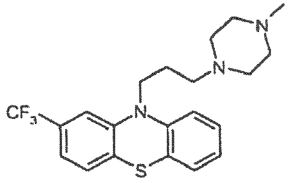
- thioridazine of formula: 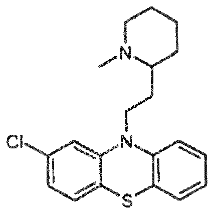 or one of their pharmaceutically acceptable salts, solvates or hydrates.

9. A compound for use according to one of claims 1 and 3 to 8, or use according to one of claims 2 to 8, **characterized in that** the compound is selected from among amitriptyline in particular the hydrochloride form, cyclobenzaprine, desipramine in particular the hydrochloride form, imipramine in particular the hydrochloride form, trimipramine in particular the maleate form, methotrimeprazine in particular the maleate form, chlorprothixene in particular the hydrochloride form or trimeprazine in particular the tartrate form.

10. Compounds for use according to one of claims 1 and 3 to 9 for the treatment or prevention of Duchenne muscular dystrophy.

11. The use according to one of claims 2 to 9, **characterized in that** the drug is for the treatment or prevention of Duchenne muscular dystrophy.

12. A compound for use according to one of claims 1 and 3 to 10, or use according to one of claims 2 to 9 and 11, **characterized in that** the said compound allows an increase in the serotonin level in the body of a living being, and in particular in man.

13. Compounds for use according to one of claims 1 and 3 to 10, or use according to one of claims 2 to 9 and 11, **characterized in that** the said compound does not allow an increase in the serotonin level in the body of a living being, and in particular in man.
